# EUROPEAN PATENT SPECIFICATION

(11) **EP 1 880 742 B1**
(45) Date of publication and mention of the grant of the patent: **31.05.2017**
(21) Application number: 06731176.1
(22) Date of filing: 05.04.2006
(51) Int. Cl.: A61M 25/10

(54) **CATHETER**
KATHETER
CATHETER

(30) Priority: 06.04.2005 JP 2005109585
(43) Date of publication of application: 23.01.2008
(73) Proprietor: Kaneka Corporation, Osaka (JP)
(72) Inventor: SHIMADA, Tamotsu, Kaneka Corporation, Settsu-shi, Osaka 5660072 (JP); TAKATERA, Masayuki, Kaneka Corporation, Settsu-shi, Osaka 5660072 (JP); NISHIDE, Takuji, Kaneka Corporation, Settsu-shi, Osaka 5660072 (JP)
(74) Representative: Gille Hrabal
(86) International application number: PCT/JP2006/307228
(87) International publication number: WO 2006/109649

(56) References cited:
- EP-A1- 0 339 093
- EP-A1- 0 365 993
- EP-A2- 0 987 045
- WO-A2-03/035159
- JP-A- 10 179 749
- JP-A- 2001 276 228
- JP-A- 2001 520 085
- JP-A- 2004 147 737
- US-A- 5 876 375

## Description

### TECHNICAL FIELD

The present invention relates to a catheter for use in medical application, and in particular, to a balloon catheter for use in peripheral angioplasty and percutaneous transluminal angioplasty (PTA, or percutaneous transluminal coronary angioplasty, PTCA) during coronary angioplasty, valve repair, or the like, a penetrating catheter for penetration through a stricture site, an injection catheter allowing administration of a treatment substance to local site, and the like.

### BACKGROUND ART

Percutaneous transluminal angioplasty has been practiced widely, for example, for treatment of stricture or obstruction of vascular lumen by enlargement and for recovery or improvement of blood flow in coronary and peripheral blood vessels. The balloon catheter used in the percutaneous transluminal angioplasty has a shaft and a balloon expandable and contractable freely by regulation of its internal pressure that is connected to the terminal region of the shaft, and the shaft generally has a structure in which a lumen (guide wire lumen) for inserting a guide wire and a lumen (inflation lumen) for supplying a pressurized fluid for regulation of the balloon internal pressure are formed in the shaft in the length direction.

The PTCA by using such a balloon catheter is generally practiced as follows: First, a guide catheter is inserted from a puncture site such as femoral artery, brachial artery, or radial artery, while the distal end is fed via aorta into the entrance of coronary artery. Then, a guide wire inserted in the guide wire lumen is fed beyond the stricture site of coronary artery, and a balloon catheter is then inserted along the guide wire, while the balloon is delivered to the stricture site. Then, the stricture site is dilated and treated by expansion of the balloon by supply of a pressurized fluid via the inflation lumen by a device such as an inflation device. After completion of the treatment of the stricture site by dilation, the PTCA is completed by contracting the balloon under reduced pressure and withdrawing it out of the body.

In the cases of a lesion significantly higher in the degree of stricture or of chronic complete obstruction, it is occasionally difficult to treat the lesion by advancing the guide wire beyond the stricture site. In such a case, a penetrating catheter is used, and the guide wire is sent beyond the stricture site.

It is often necessary to administer a therapeutic substance locally to the stricture site during PTCA. An example thereof is the treatment for dissolving thrombus by local administration of a thrombolytic agent such as urokinase. In such a case, an injection catheter is used for local administration of the therapeutic substance.

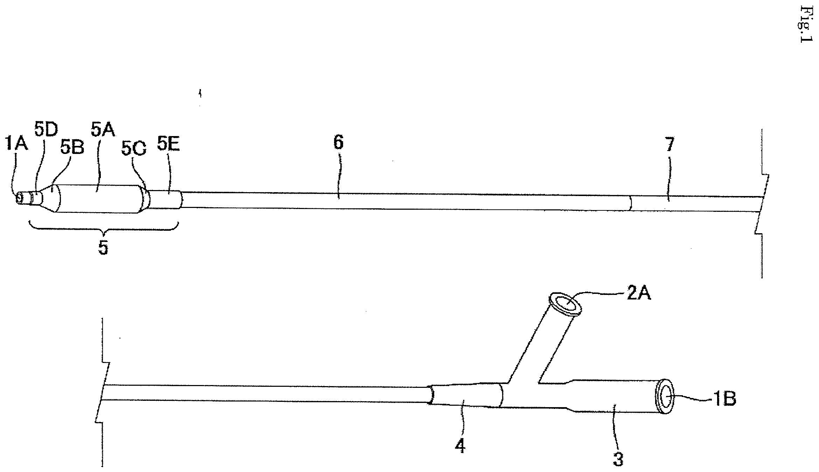

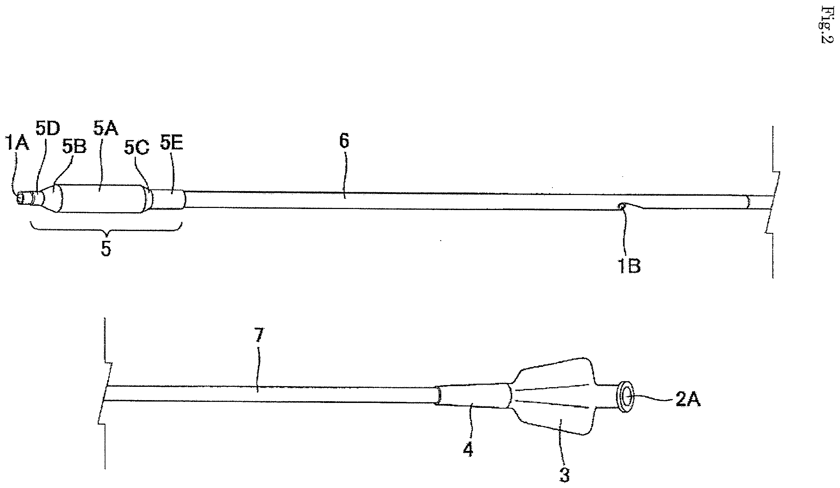

Each catheter described above has a structure in which a distal shaft and a proximal shaft are connected to each other and a hub holding the catheter is connected to the proximal end of the proximal shaft, and such catheters are divided roughly into two groups, depending on the length of the guide wire lumen. Hereinafter, common balloon catheters having a balloon connected to the distal side of the distal shaft and a port for supplying a pressurized fluid to the inflation lumen for regulation of the internal pressure of the balloon at a hub will be described as examples (Figures 1 and 2).

One is an over-the-wire catheter (OTW catheter) having a guide wire lumen formed over the entire length of a catheter as shown in Figure 1, wherein a proximal end-sided opening 1B of guide wire lumen and an opening 2A of inflation lumen are formed in a hub 3, a strain relief 4 for control of flexibility in the axial direction is also formed on the hub 3, and a distal end-sided opening 1A of the guide wire lumen is formed in the most distal end region of a balloon 5 or to the distal side of the most distal end region of the balloon 5. Another example thereof is a rapid exchange catheter (RX catheter) shown in Figure 2, in which a guide wire lumen is present only in the distal side of balloon catheter and a proximal opening 1B of the guide wire lumen is formed in the middle of the distal shaft. Because the OTW catheter has a guide wire lumen over the entire length of the balloon catheter, it is often used for sending a guide wire to the lesion that prohibits passage of the guide wire, but the operation of withdrawing the balloon catheter while leaving the guide wire in the lesion is rather complicated and causes problems. Thus, the OTW catheter demands additional special device and operation such as insertion of an exchange extension wire for withdrawal of the balloon catheter while the guide wire is left in the lesion.

On the other hand in the RX catheter, the guide wire lumen is present only in the distal side of the balloon catheter; thus, the convenience of operation is very favorable, as it is possible to remove, exchange, reinsert the balloon catheter easily while leaving the guide wire in the lesion; and it is also possible to shorten the surgical period and reduce the number of devices used.

Exemplified above are balloon catheters having a balloon in the distal side of the distal shaft, but the characteristics of the OTW and RX catheters are not limited to the balloon catheter, and are also common to penetrating catheters for stricture penetration, injection catheters for administration of a therapeutic substance, and other catheters.

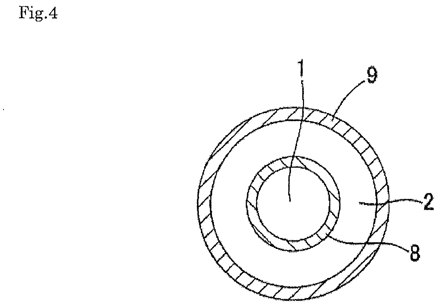

Catheters are roughly grouped into two groups, depending on the shaft structure of the region having the guide wire lumen. One is a coaxial catheter, as shown by the crosssectional shape in the Figure 4, having an internal shaft 8 and an external shaft 9 coaxially surrounding the internal shaft 8, forming a guide wire lumen 1 by the lumen of the internal shaft 8 and a second lumen ("2" in Figure 4) having a circular crosssectional shape between the internal shaft 8 and the external shaft 9. When the catheter is a balloon catheter, the second lumen is an inflation lumen, and when the catheter is an injection catheter, the second lumen is an infusion lumen. The other is a biaxial catheter wherein a guide wire lumen and a second lumen are arranged in parallel with each other (not shown in figure). Similarly in the biaxial catheter, the second lumen is an inflation lumen when the catheter is a balloon catheter, and the second lumen is an infusion lumen when the catheter is an injection catheter.

In the case of an OTW catheter, the catheter generally has a coaxial or biaxial structure over the entire length. Alternatively in the case of an RX catheter, the distal shaft in the region of guide wire lumen may have a coaxial or biaxial structure.

Various methods were disclosed for improving the convenience of operating catheter.

Japanese Patent No. 3583460 discloses an over-the-wire catheter having a first tubular part forming a guide wire lumen, a second tubular part extending in the same direction in parallel with the first tubular and having the external face bound to the peripheral surface of the first tubular part, forming an expansion lumen, and a means of changing the flexibility of at least one of the balloon and the first and second tubular parts.
According to this prior art, in the case of a catheter having biaxial shafts described above, it is possible to select the properties of the catheter properly and thus to obtain a catheter with favorable properties, by optimizing the respective components for the catheter having the first and second tubular parts and the part controlling flexibility. It is also possible to reduce cost, because the production method is simpler. However, such a catheter demands an adhesive or sleeve parts for connection of the first and second tubular parts, which lead to increase in the diameter and decrease of the flexibility of the connecting region, and thus, was not sufficiently favorable in the convenience of inserting the catheter into bent blood vessel.

Japanese Patent No. 3399556 discloses a long catheter having an internal tubular part, an external tubular part, and an expandable balloon, wherein there is a second lumen formed between the internal and external tubular parts, the expandable balloon communicates with the second lumen, the catheter has an adhesion region for adhesion between the internal and external tubular parts, the adhesion region occupies at least 30% of the internal face of the external tubular part, and the internal face of the catheter is adhered to the external face of the internal tubular part.

According to this prior art, by connecting the adhesion region of its external tubular part to the external face of the internal tubular part, it is possible to eliminate the crosssectional shape at least in one direction and thus to reduce the diameter in the region. In addition, mutual support between the internal and external tubular parts in the adhesion region improves the pressure transmission efficiency of the catheter. However, the adhesion region, where the internal and external tubular parts are bonded to each other, is lower in flexibility, and thus, it was difficult to advance the catheter by pushing the adhesion region through a bent blood vessel. The catheter also had a problem that the second lumen in the adhesion region was vulnerable to deformation when the adhesion region was bent, and the enlargement-contraction response of the expandable balloon was lower.

Japanese Patent No. 2960114 discloses a rod-shaped catheter having a balloon, i.e., a coaxial expandable balloon catheter, wherein the shaft of the catheter consists of an internal tube and an external tube surrounding the internal tube, there is an expandable lumen formed between them, the internal tube is connected to the external tube at the position distal from the proximal end region of the shaft.

In a catheter having a shaft of coaxial tubes and a balloon connected to the distal end region of the tubes, the coaxial tubes may be deformed into the nested pattern when an increased resistance is applied. The balloon deforms into an accordion-like shape by deformation in a nested pattern, making the balloon more resistant to passage through a stricture site. According to this prior art, the pressure transmission efficiency of catheter is increased, by connection of the distal end region of the external tube to the internal tube. The length of the balloon in the axial direction is kept constant for prevention of the deformation of the tube in the nested pattern. It is thus possible to prevent accordion-like deformation of the catheter and retain its favorable balloon-inserting efficiency in the stricture site. However, also in this prior art, the region where the external and internal tubes are connected is lower in flexibility, lowering the efficiency of sending the region through a bent blood vessel. In addition when the region is bent, the expandable lumen in the region deforms more easily, undesirably lowering the enlargement-contraction response of the catheter.

EP 0 987 045 A discloses relates to a balloon dilatation catheter that may be used for percutaneoustransluminal coronary angioplasty. The balloon dilatation catheter includes a coaxial construction of an inner tube and an outer tube.

WO 03/035159 A pertains to angioplasty catheters with a support block, whereby the angioplasty catheter comprises an inner tube having a proximal end, a distal end, and a lumen extending therethrough; an outer tube disposed over the inner tube, the outer tube having a proximal end and a distal end; a balloon coupled to the distal end of the outer tube; an inflation lumen defined between the inner tube and the outer tube, the inflation balloon in fluid communication with the balloon; and a support block coupled to the inner tube. In addition, a method for manufacturing an angioplasty catheter is disclosed.

### DISCLOSURE OF THE INVENTION

### Technical Problems to be Solved

Under the circumstances above, an object of the present invention is to provide a catheter having a coaxial shaft structure in the region of guide wire lumen, having favorable pressure transmission efficiency allowing transmission of the force applied to the catheter proximal end side efficiently to the distal side or distal end of the catheter, and having favorable insertion efficiency allowing insertion of the catheter into stricture site while the deformation of the internal and external shafts for the coaxial structure in a nested pattern is prevented, without deterioration in the flexibility of catheter, and also in the enlargement-contraction response of the balloon in the case of a balloon catheter and in injection response in the case of an injection catheter.

### Means to Solve the Problems

After intensive studies to solve the problems above, the inventors have invented a catheter (25), comprising:
proximal (7) and distal (6) shafts respectively having proximal and distal end regions, wherein the distal end of the proximal shaft (7) and the proximal end of the distal shaft (6) are connected to each other, the proximal end of the proximal shaft (7) is connected to a hub (3) for holding the catheter (25),
at least part of the distal shaft (6) has an internal shaft (8) and an external shaft (9) enclosing the internal shaft (8) coaxially, the internal shaft (8) extends out of the external shaft (9) toward the distal side, the lumen of the internal shaft forms a guide wire lumen,
a projection (12) formed on an external surface of said internal shaft (8) and a diameter-reducing protuberance (13) formed on an internal surface of said external shaft (9) are in contact with each other, so as to prohibit the relative movement of the external (9) and internal (8) shafts when an insertion force at a particular intensity or more is applied to the catheter,
said projection is placed to the distal side of said diameter-reducing protuberance (13), wherein said projection (12) and diameter-reducing protuberance (13) are provided at the proximal end of a balloon-expandable region.

The catheter according to the present invention is a catheter having an additional foldable balloon with a proximal end region and a distal end region, wherein the proximal end region is connected to the distal region of the external shaft, the distal end region is connected to the distal region of the internal shaft, there is an inflation lumen having a circular crosssectional shape formed between the internal and external shafts, and the inflation lumen communicates with the space in the balloon, and, there is an inflation lumen formed securely in the contact region, in the state where the projection and the diameter-reducing protuberance are in contact with each other and relative movement of the external shaft and the internal shaft is restricted.

The catheter according to the present invention may be a penetrating catheter for penetration in the stricture site of body cavity, wherein the distal region of the internal shaft and the distal region of the external shaft are connected to each other.

Alternatively, the catheter according to the present invention may be an injection catheter allowing administration of a therapeutic substance to a local site in the body cavity, wherein the distal region of the internal shaft and the distal region of the external shaft are connected to each other, there is an infusion lumen having a circular crosssectional shape formed between the internal and external shafts, and there is an injection hole formed on the external shaft, and preferably in such a case, the infusion lumen is formed securely in the contact region, in the state where the projection and the diameter-reducing protuberance are in contact with each other and relative movement of the external shaft and the internal shaft is restricted.

### Effects of the Invention

The present invention provides a catheter having a coaxial shaft structure in the region of guide wire lumen, having pressure transmission efficiency transmitting the force applied to the catheter proximal end side efficiently to the distal side or distal end of the catheter, and having favorable insertion efficiency into stricture site while the deformation of the internal and external shafts for the coaxial structure in a nested pattern is prevented, without deterioration in the flexibility of catheter, and also in the enlargement-contraction response of the balloon in the case of a balloon catheter and in injection response in the case of an injection catheter.

### BRIEF DESCRIPTION OF THE DRAWINGS

Figure 1 is a schematic perspective view illustrating an over-the-wire catheter (OTW catheter), among common balloon catheters.
Figure 2 is a schematic perspective view of a rapid exchange catheter (RX catheter), among common balloon catheters.
Figure 3 is a partial schematic side view illustrating the cross section of the distal region of a common RX balloon catheter having a guide wire lumen region in the coaxial structure.
Figure 4 is an A-A' sectional view of the catheter shown in Figure 3.
Figure 5 is a partial schematic side view showing the vertical cross section of a common RX catheter balloon catheter, when the balloon deforms into an accordion-like shape.
Figure 6 is a partial schematic side view showing the vertical cross section of a balloon catheter according to the present invention.
Figure 7 is an example of the B-B' sectional view of the catheter shown in Figure 6.
Figure 8 is another example of the B-B' sectional view of the catheter shown in Figure 6.
Figure 9 is yet another example of the B-B' sectional view of the catheter shown in Figure 6.
Figure 10 is yet another example of the B-B' sectional view of the catheter shown in Figure 6.
Figure 11 is an example of the C-C' sectional view of the catheter shown in Figure 6.
Figure 12 is an example of the C-C' sectional view of the catheter shown in Figure 6.
Figure 13 is another example of the C-C' sectional view of the catheter shown in Figure 6.
Figure 14 is another example of the C-C' sectional view of the catheter shown in Figure 6.
Figure 15 is yet another example of the C-C' sectional view of the catheter shown in Figure 6.
Figure 16 is yet another example of the C-C' sectional view of the catheter shown in Figure 6.
Figure 17 is yet another example of the C-C' sectional view of the catheter shown in Figure 6.
Figure 18 is a partial schematic side view showing the vertical cross section of the projection 12 and the diameter-reducing protuberance 13 in contact with each other shown in Figure 6.
Figure 19 is a partial schematic side view illustrating the vertical cross section of an injection catheter according to the present invention.
Figure 20 is a partial schematic side view illustrating the vertical cross section of another injection catheter according to the present invention.
Figure 21 is a schematic perspective view illustrating an example of a core material used in processing an example of the diameter-reducing protuberance according to the present invention.
Figure 22 is another schematic perspective view illustrating an example of a core material used in processing an example of the diameter-reducing protuberance according to the present invention.
Figure 23 is a schematic view illustrating an evaluation system used in evaluation of the catheter according to the present invention.

### EXPLANATION OF REFERENCES

- 1: Guide wire lumen
- 1A: Distal end-sided opening of guide wire lumen
- 1B: Proximal end-sided opening of guide wire lumen
- 2: Inflation lumen
- 2A: Inflation lumen opening
- 3: Hub
- 4: Strain relief
- 5: Balloon
- 5A: Straight tube region
- 5B: Distal tapered region
- 5C: Proximal tapered region
- 5D: Distal connecting region
- 5E: Proximal connecting region
- 6: Distal shaft
- 7: Proximal shaft
- 8: Internal shaft
- 9: External shaft
- 10: Infusion lumen
- 11: X-ray impermeable marker
- 12: Projection
- 13: Diameter-reducing protuberance
- 14: Fin
- 15: Tubular part
- 16: Injection hole
- 17: Second lumen
- 18: Simulated stricture site
- 19: Simulated blood vessel
- 20: Slide table
- 21: Guide wire
- 22: Catheter-holding unit
- 23: Force gauge
- 24: Hole
- 25: Catheter
- 26A and 26B: Core material
- 27a, 27b, 27C, and 27d: Cylindrical region

### BEST MODE FOR CARRYING OUT THE INVENTION

Hereinafter, favorable embodiments of the catheter according to the present invention will be described in detail with referring to the drawings, by taking a case where the catheter is a balloon catheter as the main example.

The catheter according to the present invention is a coaxial catheter having proximal and distal shafts respectively having proximal and distal end region, wherein the distal end of the proximal shaft and the proximal end of the distal shaft are connected to each other, the proximal end of the proximal shaft is connected to a hub for holding the catheter, at least part of the distal shaft has an internal shaft and an external shaft enclosing the internal shaft coaxially, the internal shaft extends out of the external shaft toward the distal side, and the lumen of the internal shaft forms a guide-wire lumen, and the other structure is not particularly limited. Thus, the catheter may be an OTW catheter shown in Figure 1 or an RX catheter shown in Figure 2. Alternatively, it may have the other structure.

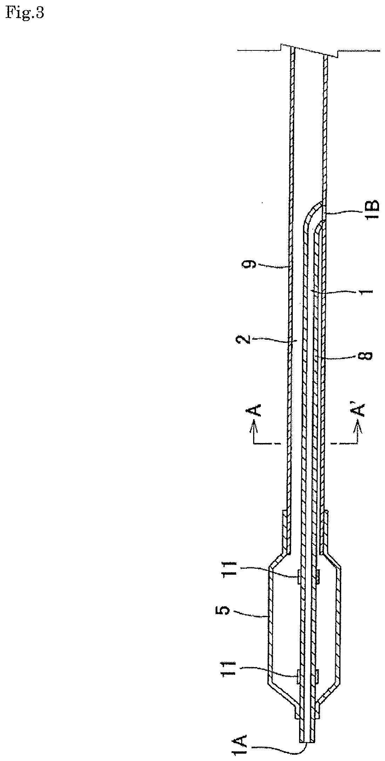

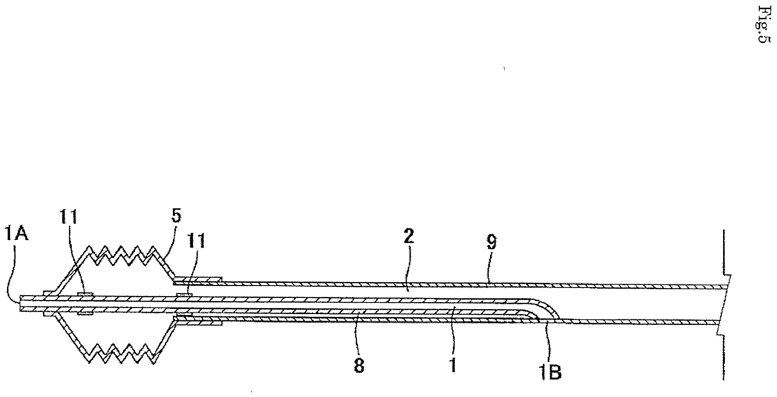

In the case of a typical RX balloon catheter having a coaxial shaft such as that shown in Figure 3, the cross section A-A' in the coaxial region has the structure shown in Figure 4. To place a catheter such as that shown in Figure 3 at a desirable treatment area it is necessary to push the catheter forward by applying force to the hub formed at the proximal end of the proximal shaft. When the route to the treatment area is bent in complicated way and the degree of stricture in the treatment area is high, the resistance of the catheter becomes higher, demanding greater force. In such a case, the internal shaft 8 and the external shaft 9, which constitute at least part of the distal shaft, deform in a nested pattern, and, as a result, the balloon 5 region may be deformed like accordion as schematically shown in Figure 5. Such deformation increases the external diameter of the balloon 5 region, making the placement thereof in the treatment area more difficult.

Catheters other than balloon catheter, such as penetrating catheter for penetration beyond stricture site and injection catheter for administration of a therapeutic substance, do not have a balloon and thus, do not show accordion-like deformation in the balloon region as described above, but it is difficult to transmit the force to the catheter distal end sufficiently, because the internal and external shafts bind to each other in a nested pattern. Thus, it is not easy to place such a catheter properly in the treatment area.

In contrast, the catheter according to the present invention is characterized by having a means to prohibit the relative movement of the external and internal shafts when an insertion force at a particular intensity or more is applied. The means prevents the phenomenon of the external and internal shafts binding to each other in a nested pattern and thus the accordion-like deformation in the balloon region effectively. It is also possible to transmit the force applied to the hub to the catheter distal end effectively and operate the catheter in the treatment area more conveniently.

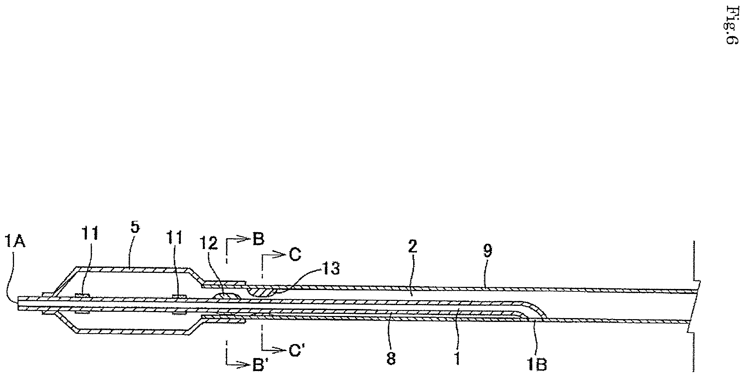

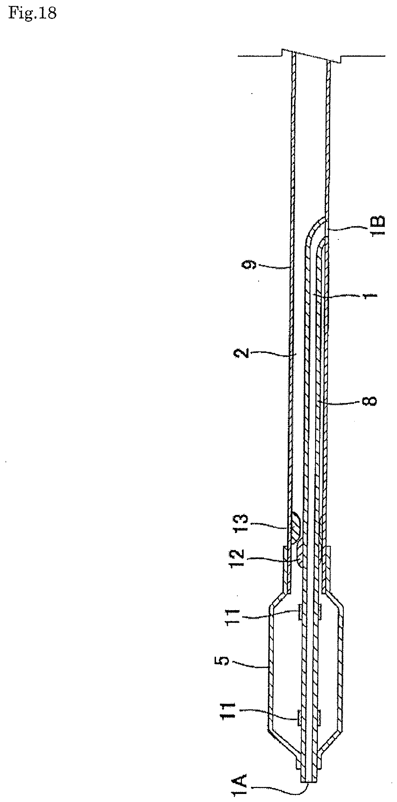

Considering the flexibility of the region containing the means and the reliability of the step forming the means, the means have a projection 12 formed on the external face of the internal shaft 8 and a diameter-reducing protuberance 13 formed on the internal face of the external shaft 9, as shown in Figure 6. When the means consisting of the projection 12 and the diameter-reducing protuberance 13 is present, if force is applied to the catheter, the projection 12 and the diameter-reducing protuberance 13 contact with each other, preventing further fitting between the external shaft 9 and the internal shaft 8 in a nested pattern, as shown in Figure 18. The structure of the region when the diameter-reducing protuberance 13 and the projection 12 contact with each other will be described below, but is not limited thereto, and any modifications in the scope of the present invention that are possible by those who are skilled in the art are also included in the structure. When the projection and the diameter-reducing protuberance are in direct and/or loose contact with each other (including the case where the inflation lumen is not deformed to a degree that it is completely closed when both of them (or one of them) are in contact), the concept that "the projection and the diameter-reducing protuberance contact with each other" include the case where the projection and the diameter-reducing protuberance fit to each other.

In the prior art described in Patent Documents JP3399556 and JP2960114 the external and internal shafts, which are held fixed to each other consistently, caused a problem that the catheter was always lower in flexibility at the fixing site and unfavorable in the convenience of operation. However, the flexibility of the catheter according to the present invention having the means above is not damaged, because the external shaft 9 and the internal shaft 8 are not fixed to each other until an insertion force at a particular intensity or more is applied to the catheter. Even when an insertion force at a particular intensity or more is applied, the structure in which the projection 12 and the diameter-reducing protuberance 13 stop moving in contact with each other is more flexible and more convenient for operation than the structure described in Patent Documents JP3399556 and JP2960114 in which the external and internal shafts are bound to each other consistently.

In the present invention, the relative location of the projection 12 and the diameter reducing protuberance 13 varies according to the kinds of the resins forming the external and internal shafts and the structure of the catheter, but, for example, if a greater pressure is applied to the internal shaft than to the external shaft when the catheter is pushed, and as a result, the internal shaft moves more easily, the projection 12 is preferably located to the distal side of the diameter-reducing protuberance 13 (e.g., Figures 6, 19, and 20). In such a case if the projection 12 is located to the distal side, when an insertion force at a particular intensity or more is applied to the catheter, the projection 12 moves relatively to the proximal end side and stops in contact with the diameter-reducing protuberance 13, as shown in Figure 18. Alternatively when a great pressure is applied to the external shaft than to the internal shaft of the catheter and thus, the external shaft moves relatively more, the diameter-reducing protuberance 13 is located to the distal side of the projection 12.

The relative location of the projection and the diameter-reducing protuberance is determined according to the kind of catheter and the desirable treatment area. In the case of a balloon catheter for vasodilation of the stricture site of cardiac coronary artery, the relative location of the projection and the diameter-reducing protuberance is as close to the proximal end of the balloon-expandable region as possible, in the range that does not damage the flexibility of the balloon catheter. When present in the region as close to the expandable-region proximal end as possible, the projection and the diameter-reducing protuberance contact with each other easily before deformation in the accordion shape. The proximal end of expandable region corresponds to the proximal end of the proximal tapered region 5C shown in Figure 1.

When the projection is located to the distal side of the diameter-reducing protuberance, the distance between the proximal end of the projection and the distal end of the diameter-reducing protuberance in contact with each other is determined, by considering the materials for the internal and external shafts or the dimensions of the internal and external shafts, the insertion force possibly applied to the catheter, and others. For example, in the case of a balloon catheter for vasodilation of the stricture site of cardiac coronary artery, the distance is preferably 5.0 mm or less. A distance of 5.0 mm or more is unfavorable, because it leads to deformation of the balloon region into the accordion shape before the projection and diameter-reducing protuberance contact with each other. The distance between the proximal end of the diameter-reducing protuberance and the distal end of the projection in contact with each other when the diameter-reducing protuberance is located to the distal side of the projection is also the same.

The length of the projection or the diameter-reducing protuberance in the shaft axial direction is determined, by considering the material for the internal or external shaft or the dimension of the internal or external shaft, the insertion force possibly applied to the catheter, and others. An excessive length of the projection or the diameter-reducing protuberance leads to deterioration in the flexibility of catheter and is thus unfavorable. Alternatively, an excessively shorter length of the projection or the diameter-reducing protuberance may lead to movement of the projection beyond the diameter-reducing protuberance to the proximal end side of the diameter-reducing protuberance even after they are in contact with each other, and thus is unfavorable. For example, in the case of a balloon catheter for vasodilation of the stricture site of cardiac coronary artery, the length of the projection is preferably 0.5 mm or more and 3.0 mm or less, more preferably 1.0 mm or more and 2.0 mm or less, and the length of the diameter-reducing protuberance is preferably 0.5 mm or more and 7.0 mm or less, more preferably 2.0 mm or more and 5.0 mm or less.

The catheter according to the present invention is a balloon catheter having a foldable balloon with a proximal end region and distal end region, wherein the proximal end region is connected to the distal region of the external shaft, the distal end region is connected to the distal region of the internal shaft, an inflation lumen having a circular cross section is formed between the internal and external shafts, and the inflation lumen communicates with the area inside the balloon. In such a balloon catheter, the inflation lumen 2 in the contact region is preferably preserved securely in the state that the projection 12 is in contact with the diameter-reducing protuberance 13 and the relative movement of the external shaft 9 and the internal shaft 8 is restricted, as shown in Figure 18. The contact region is a region where the projection 12 and the diameter-reducing protuberance 13 are in contact with each other (the same shall apply hereinafter). The phrase that the inflation lumen 2 is preserved securely indicates that the inflation lumen 2 is not enclosed or blocked completely in the contact region. Thus by preserving the inflation lumen securely, it becomes possible to dilate the balloon in a desirable treatment area, even when the projection and the diameter-reducing protuberance become in contact with each other by the insertion force applied to the catheter for placement of the balloon catheter in the desirable treatment area. Typical structures of the projection and the diameter-reducing protuberance will be described below.

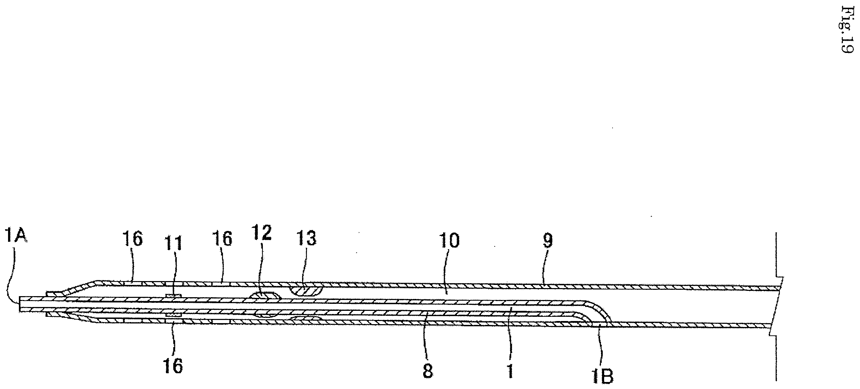

The catheter according to the present invention is an injection catheter allowing administration of a therapeutic substance to a local site in the body cavity, as shown in Figure 19, having an injection hole 16 in the external shaft 9, wherein the distal region of the internal shaft 8 is connected to the distal region of the external shaft 9, and an infusion lumen 10 having a circular cross section is formed between the internal shaft 8 and the external shaft 9. In the case of such an injection catheter, the infusion lumen 10 is preferably preserved securely in the contact region, in the state where the projection 12 and the diameter-reducing protuberance 13 are in contact with each other and relative movement of the external shaft 9 and the internal shaft 8 is restricted. The phrase that the inflation lumen is preserved securely indicates that the inflation lumen is not enclosed or blocked completely in the contact region. It is possible to administer a therapeutic substance to treatment area by preserving the infusion lumen 10 securely as described above, even when the projection 12 and the diameter-reducing protuberance 13 are brought into contact with each other by the insertion force applied to the catheter for placing an injection catheter in a desirable treatment area. Typical structure of the projection and the diameter-reducing protuberance will be described below.

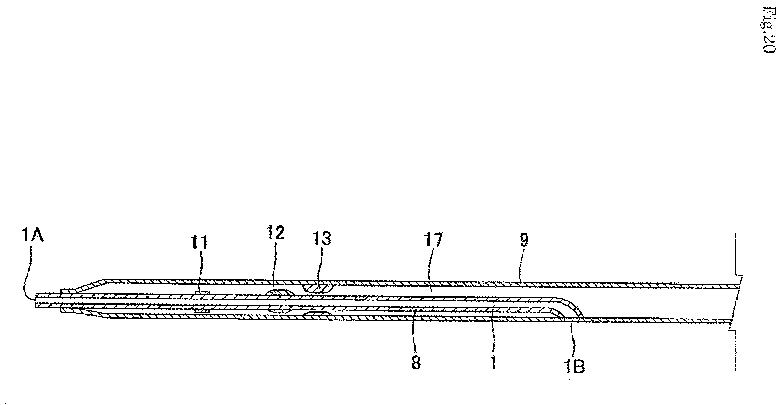

Alternatively, the catheter according to the present invention may be a penetrating catheter for penetration into the stricture site of body cavity having its distal region of the internal shaft 8 and the distal region of the external shaft 9 connected to each other, such as that shown in Figure 20. In the case of such a penetrating catheter, the lumen having a circular cross section between the internal shaft 8 and the external shaft 9 does not have any function, and thus, the lumen needs not to be preserved securely in the contact region in the state where the projection 12 and the diameter-reducing protuberance 13 are in contact with each other and relative movement of the external shaft 9 and the internal shaft 8 is restricted. However, the lumen may be preserved for improvement of the flexibility of the area close to the contact region.

In the present invention, the structure of the projection 12 formed on the internal shaft 8 and the diameter-reducing protuberance 13 formed on the external shaft 9 is not particularly limited, if it is the one that the external shaft 9 and the internal shaft 8 stop in contact with each other before the catheter becomes in the nested state, as they move relatively to each other, as shown in Figure 18. As described above, when the catheter is a balloon catheter or an injection catheter (Figure 6 or 19), it preferably has a structure having the inflation lumen 2 or the infusion lumen 10 securely when the projection 12 and the diameter-reducing protuberance 13 are in contact with each other. If the catheter is a penetrating catheter (Figure 20), the projection 12 and the diameter-reducing protuberance 13 may be in the structure forming or not forming a second lumen 17 as they become in contact with each other.

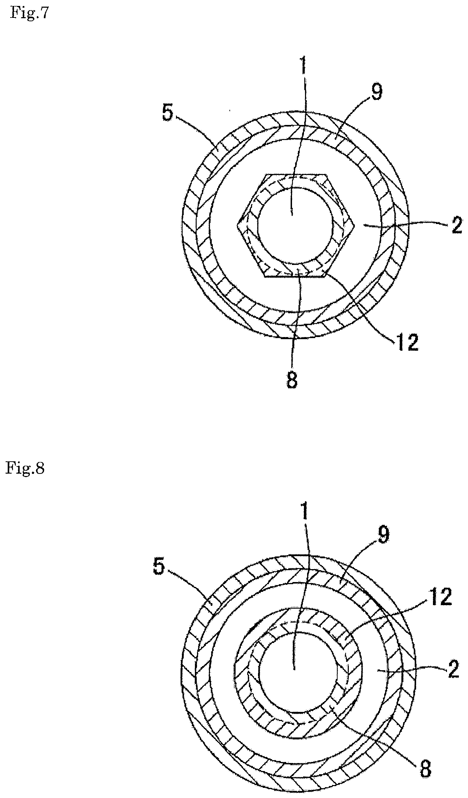

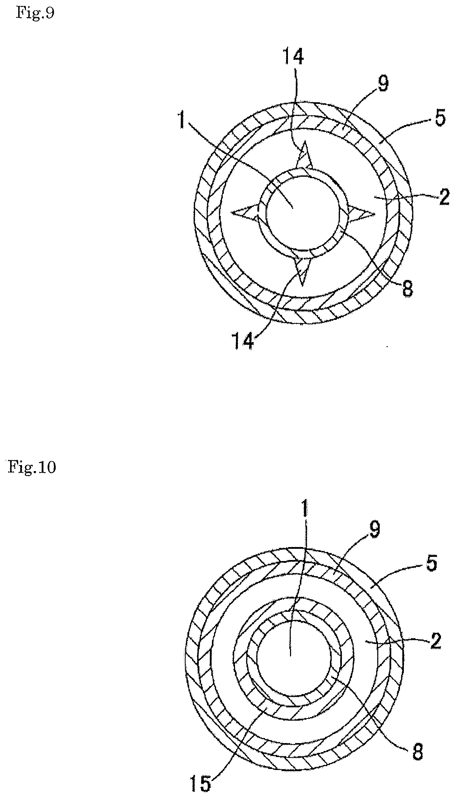

Favorable examples of the structure of the projection 12 above are shown in Figures 7 to 10. Each structure shown corresponds to the B-B' cross section in Figure 6. The projections 12 are formed by making a region having an external diameter larger than that of the tubular internal shaft 8 to a degree that the projection becomes in contact with the diameter-reducing protuberance 13, specifically by making the external shape of the internal shaft 8 hexagonal in Figure 7, by increasing the thickness of the internal shaft 8 while keeping the internal diameter thereof constant in Figure 8, by forming four fins 14 on the external face of the internal shaft 8 in Figure 9, or by connecting a tubular part 15 to the external face of the internal shaft 8 in Figure 10. For example in Figures 7 and 8 the regions larger in size than the tubular cross section of the internal shaft 8 itself represent the projections 12. Alternatively, the fin 14 shown in Figure 9 or the tubular part 15 shown in Figure 10 represents the projection 12 as it is. These are only some of favorable examples, and various other shapes may be used, if the shape allows contact with the diameter-reducing protuberance 13. When the shape of the projection is smaller (e.g., as the fin shown in Figure 9), it is possible to obtain a favorable area of the inflation or infusion lumen and make liquid flow through the lumen favorably.

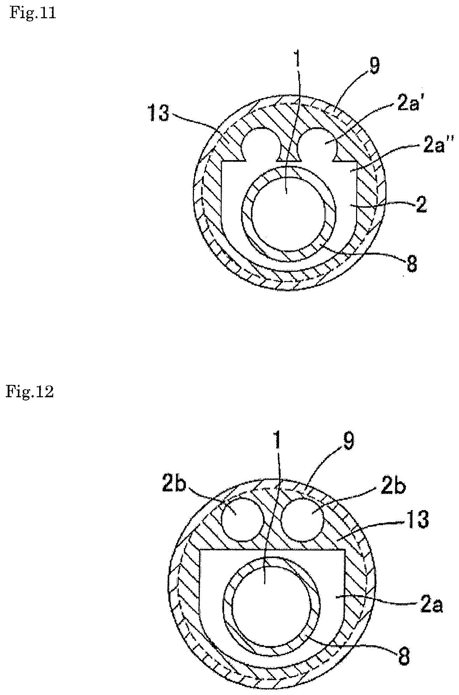

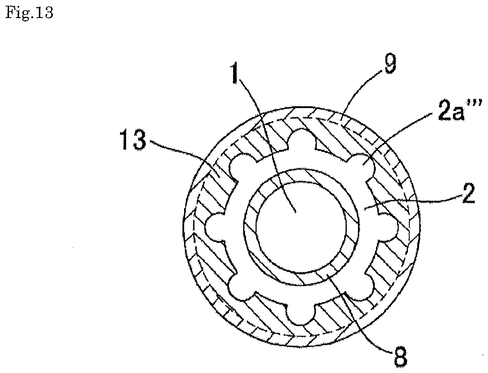

Examples of the favorable structures of the diameter-reducing protuberance 13 are shown in Figures 11 to 17. Each structure corresponds to the C-C' cross section in Figure 6. For example when the catheter is a balloon catheter, there is formed a diameter-reducing protuberance 13 having an inflation lumen 2 in the shape in combination of semicircular, quadrangular and circular forms or in the gear shape in Figure 11 or 13. It is possible to keep liquid flow into the inflation lumen 2 to a favorable degree, by making the shape different from the circular form, even when the diameter-reducing protuberance 13 is in contact with the projection 12. For example, in Figure 11, the inflation lumen 2 has two semicircular region 2a' and a quadrangular region 2a". In Figure 13, the inflation lumen 2 has a gear-shaped semicircular region 2a"'.

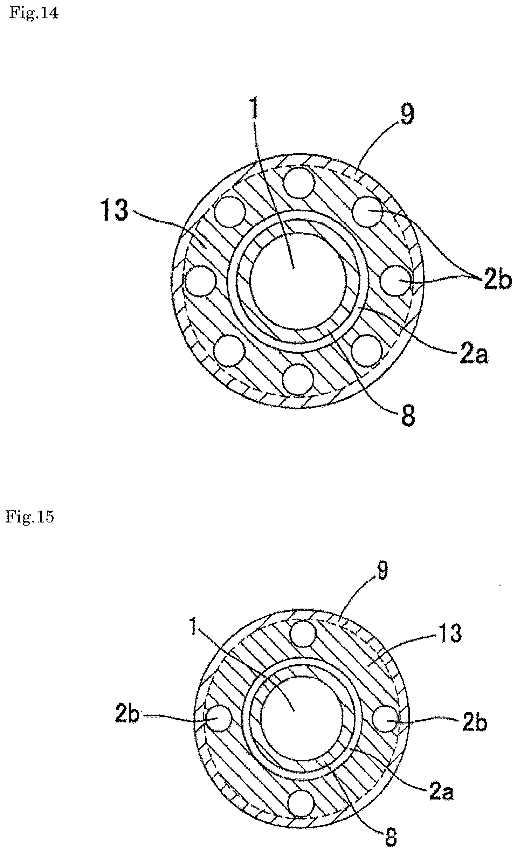

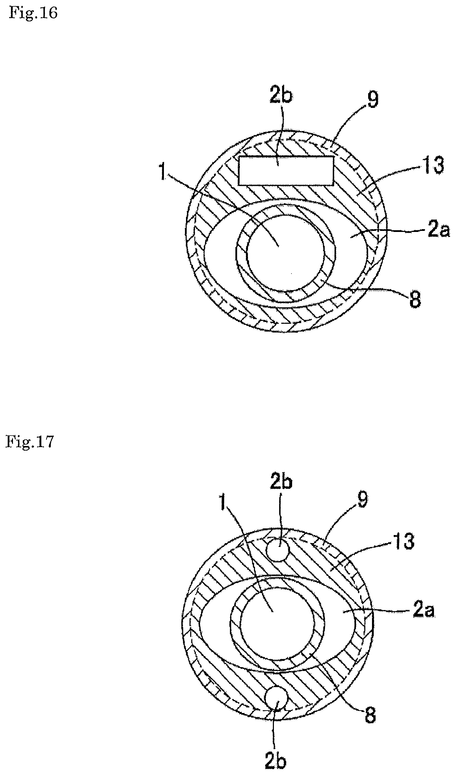

In Figures 12, 14, 15, 16, and 17, the diameter-reducing protuberance 13 has a circular lumen 2a enclosing an internal shaft 8 and also an inflation lumen 2b independently, and the shape and the number of the inflation lumens 2b are different. In such a case, it is possible to adjust the liquid flow rate to a favorable degree by controlling the inflation lumen 2b. As for the shape and the number, the catheter has two circular inflation lumens 2b in Figure 12, eight circular inflation lumens in Figure 14, four circular inflation lumens in Figure 15, one rectangular inflation lumen in Figure 16, or two circular inflation lumens in Figure 17. It is possible in any case to bring the diameter-reducing protuberance 13 into contact with the projection 12, and thus, to retain its favorable inflation lumen 2 securely by making liquid flow favorable even during they are in contact with each other, because the inflation lumens 2a and 2b are communicating with each other in an area other than the contact region. These are only some of favorable examples, and various other shapes not shown in the Figures may be used.

Even in the case of a penetrating or injection catheter, the structures shown in Figures 11 to 17 are used favorably as the means of controlling relative movement of the internal and external shafts. In particular in the case of a penetrating catheter, in addition to the structures shown in Figures 11 to 17, a structure having no inflation lumen (in the case of balloon catheter) or a structure having no infusion lumen (in the case of injection catheter) is also used favorably.

The method of forming the projection on the external face of internal shaft is not particularly limited. Examples thereof include, a method of connecting a tubular part 15 to the external face of an internal shaft 8 having a circular cross section as shown in Figure 10, a method of forming a tube having a crosssectional shape shown in Figures 7 to 9 corresponding to the internal shaft 8, for example, by extrusion molding, a method of forming a crosssectional shape shown in Figure 8 by connecting a terminal-enlarged tube to another tube, and the like.

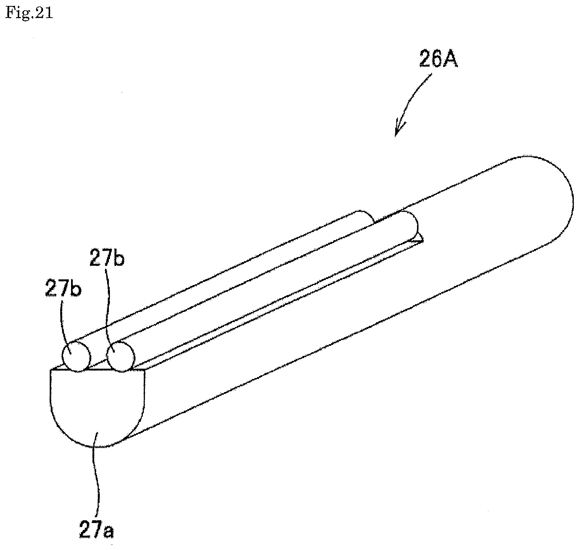

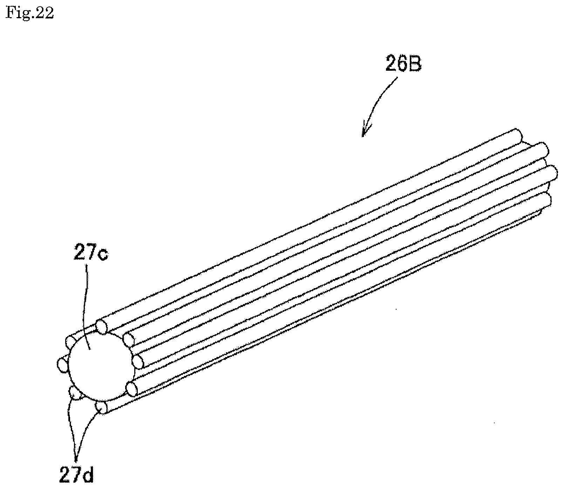

Similarly, the method of forming the diameter-reducing protuberance on the external shaft is not particularly limited. In the case of the structure in which the crosssectional shape of the inflation lumen 2 is modified, as shown in Figure 11 or 13, it is possible to form a lumen in the shape above by inserting for example core materials 26A and 26B shown in Figure 21 or 22 into the external shaft and heat-treating the composite. As shown in Figures 12, 14, 15, 16, and 17, in the case of a structure wherein the circular lumens 2a and the inflation lumen 2b surrounding the internal shaft 8 are formed independently, a tube having a desirable crosssectional shape may be prepared for example by extrusion molding and connected to the external shaft.

The method of connecting the internal or external shaft to another tube in preparation of the projection or the diameter-reducing protuberance is, for example, adhesion with an adhesive, fusion if the material is a combination of thermoplastic materials, and others. When an adhesive is used, the composition, chemical structure, and hardening mode thereof are not limited. Thus, adhesives such as of a urethane, silicone, epoxy, or cyanoacrylate resin are used favorably from the points of composition and chemical structure, and two-liquid mixing, UV-hardening, water hardening, thermally hardening adhesives are used favorably from the point of hardening mode. When an adhesive is used, use of an adhesive having a post-hardening hardness prohibiting discontinuous change in rigidity in the region before and after the connection region is preferable, and the adhesive may be selected, for example, according to the material, dimension, and rigidity of the connection region. The connection region may be heat-treated for reduction in diameter of the connection region, and if a material less adhesive, such as polyolefin, is used, the connection region may be plasma-processed before adhesion, for example with oxygen gas, for improvement in adhesiveness.

For connection by fusion, a core material in any dimension and shape may be inserted into the tube for the external shaft, for making a desirable lumen. Examples thereof include, but are not limited to, the core materials 26A and 26B respectively shown in Figures 21 and 22. In such a case, considering removal of the core material after completion of the processing, a fluorine resin such as of polytetrafluoroethylene, poly-para-xylylene, polymonochloro-para-xylylene, or the like is preferably coated on the outermost surface of the core material, for easier removal of the core material. The dimension and the crosssectional shape of the core material is not particularly limited, if the advantageous effects of the present invention are obtained, and may be determined according to the processability during processing, the crosssectional area of the desirable lumen, and the like.

The materials for the distal shafts, i.e., the internal shaft and the projections formed on the internal shaft, the external shaft and the diameter-reducing protuberance formed on the external shaft, are not particularly limited. The internal shaft (or external shaft) and the projection (or diameter-reducing protuberance) may be formed with the same material or with different materials. Examples of the materials for the internal shaft or the projection include polyolefin, polyolefin elastomers, polyester, polyester elastomers, polyamide, polyamide elastomers, polyurethane, polyurethane-elastomers, and the like. Because the lumen of the internal shaft forms a guide wire lumen, polyethylene, high-density polyethylene in particular, is preferable, considering the lubricity of the guide wire; and it is more preferable to make at least part of the internal shaft have a multi-layered structure, by forming the innermost layer with high-density polyethylene and the outermost layer with a material fusable with balloon or the external shaft. It is possible to realize the present invention easily, by using such a multilayer structure as the projection. In addition, the lumen of the internal shaft may be coated, for example, with polydimethylsiloxane for improvement in lubricity of the guide wire.

The material for the external shaft or the diameter-reducing protuberance is also not particularly limited. Examples thereof include polyolefin, polyolefin elastomers, polyester, polyester elastomers, polyamide, polyamide elastomer, polyurethane, polyurethane elastomers, and the like.

Similarly, the material for the proximal shaft is also not particularly limited, but the material for the proximal shaft preferably has a rigidity similar to or higher than that of the distal shaft. More preferable from the points of processability, compatibility with the body, and others are, for example, metals such as stainless steel, high-rigidity resin materials such as polyimide, polyamide-imide, and polyether ether ketone, and the like. For continuous distribution of the rigidity of the catheter in the length direction, the rigidity of the distal side of the proximal shaft may be made lower than that of the proximal end side of the proximal shaft, by forming spiral cut, groove, slit, or the like at the distal side of the proximal shaft.

When the catheter is an injection catheter, an injection hole 16 is preferably formed on the external shaft 9 for the distal shaft, as exemplified in Figure 19. The size, site, and the number of the injection holes 16 may be determined arbitrarily according to the properties of the therapeutic substance used. The method of preparing the injection hole 16 is also not particularly limited; machining processing, laser processing, or the like may be used; and the kind of the laser used is also not particularly limited.

When the catheter is a balloon catheter, it is produced, for example, by dipping molding or blow molding, and a suitable method is selected according to application. In the case of a balloon catheter for vasodilation of the stricture site of cardiac coronary artery, use of blow molding is preferable for sufficient pressure resistance. A method of preparing a balloon by blow molding will be described below. First, a tubular parison in arbitrary dimension is formed for example by extrusion molding. The tubular parison is placed in a metal mold having a shape corresponding to the balloon shape, forming a balloon having the shape identical with that of the mold by stretching it in the axial and diameter directions by biaxial stretching step. The biaxially stretching step may be carried out under heated condition for multiple times. The stretching in the axial direction may be preformed simultaneously with or before or after stretching in the diameter direction. The balloon may be annealed additionally for stabilization of its shape and dimension.

As shown in Figures 1 and 2, the balloon has a straight tube region 5A, connecting regions (5D and 5E) at the distal and proximal sides, and tapered regions (5B and 5C) between the straight tube region 5A and the connecting regions (5D and 5E). The dimension of the balloon is determined according to the application of the balloon catheter, but the external diameter of the straight tube region when enlarged is 1.00 mm to 35.00 mm, preferably 1.25 mm to 30.00 mm, and the length of the straight tube region is 5.00 mm to 80.00 mm, preferably 7.00 mm to 60.00 mm. When it is a balloon catheter for vasodilation of the stricture site of cardiac coronary artery, the external diameter of the straight tube region when enlarged is preferably 1.25 mm to 5.0 mm, and the length of the straight tube region is preferably 7.00 mm to 40.00 mm.

The resin for the tubular parison is not particularly limited, and examples thereof for use include polyolefin, polyolefin elastomers, polyester, polyester elastomers, polyamide, polyamide elastomers, polyurethane and polyurethane elastomers, and the like, and the blends of two or more of these resins and multilayered materials having two or more layers of these resins are also favorable.

Examples of the materials for the hub connected to the proximal end of the proximal shaft of the catheter include polycarbonate, polyamide, polyurethane, polysulfone, polyarylate, styrene-butadiene copolymers, polyolefin, and the like.

A core wire may be placed in the catheter, for improvement in convenience of the operation of inserting the catheter along the guide wire from outside, efficient transmission of the force applied to the catheter to the distal end, and prevention of catheter kink (twist). The core wire may be placed in any region of the catheter, but preferably placed in a lumen other than the guide wire lumen for convenience of operation of inserting the catheter along the guide wire. Part of the external diameter of the core wire may be tapered in the direction toward the distal end to make the distribution in rigidity of the catheter in the length direction more uniform. The core wire is not particularly limited, if it is a metal. The material for the core wire is determined according to the kind and application of the catheter, but preferably a stainless steel alloy, a cobalt-chromium alloy, or a nickel-titanium alloy, from the point of processability or compatibility with the body. The method of processing the core wire is also not particularly limited, and methods such as centerless grinding are used favorably.

An X-ray impermeable marker may be installed to make a particular unit of the catheter more visible and locate the catheter more easily during treatment with the catheter according to the present invention. The X-ray impermeable marker is not limited if it is an X-ray-impermeable material, and the kind of the material, metal or resin, is not limited. The site and the number thereof formed are also not limited, and are determined according to the application of the catheter. For example as shown in Figure 6, X-ray impermeable markers 11 may be formed at two sites on the external face of the internal shaft 8 extending in the balloon 5, or an X-ray impermeable marker 11 may be formed only at one site on the external face of the internal shaft 8, as shown in Figures 19 and 20.

The external face of the catheter may be coated, for improvement in hydrophilicity, and thus, for easier insertion of the catheter into the blood vessel or the guide catheter. Accordingly, at least part of the area in contact with blood such as of distal shaft and proximal shaft may be coated with a hydrophilic material that becomes lubricant when in contact with the blood. However, the area and the length of the hydrophilic coating are determined according to the application of the catheter. Favorable examples of the hydrophilic coating agents include hydrophilic polymers such as poly(2-hydroxyethyl methacrylate), polyacrylamide, and polyvinylpyrrolidone, and the coating method is also not particularly limited.

When the catheter is a balloon catheter, the external face of the balloon may be hydrophobically coated for prevention of slipping of the balloon during enlargement, according to application of the catheter. The kind of the hydrophobic coating is not particularly limited, and a hydrophobic polymer such as polydimethylsiloxane is used favorably.

### EXAMPELS

Hereinafter, typical Examples and Comparative Examples of the present invention will be described in detail, but it should be understood that the present invention is not limited to the following Examples.

### (Example 1)

A tubular parison (internal diameter: 0.43 mm, external diameter: 0.89 mm) was prepared by extrusion molding of a polyamide elastomer (PEBAX7033SA01, Elf Atochem); and a balloon having a straight-tube external diameter of 3.0 mm, a straight-tube length of 20 mm, a length of the tapered regions at the distal and proximal end sides of 5 mm, and a length of the connecting regions at the distal and proximal end sides of 5 mm was prepared by biaxially stretching blow molding of the parison.

A tube (internal diameter: 0.43 mm, external diameter: 0.56 mm) was prepared by extrusion molding of high-density polyethylene (HY540, Japan Polychem Corporation). The high-density polyethylene tube was cut into pieces of 46 mm and 254 mm in length. One end of the 46 mm-cut tube was expanded to an internal diameter of 0.58 mm, and one end of the 254 mm-cut tube was inserted into the expanded region and fused by heating, to give an internal shaft. The heat-fused region represents the projection.

A tube (internal diameter: 0.72 mm, external diameter: 0.86 mm) was prepared by extrusion molding of a polyamide elastomer (PEBAX7233SA01, Elf Atochem). The tube was heat-treated by using a core material 26A having the shape shown in Figure 21 (diameter of large cylindrical region 27a: 0.58 mm, diameter of small cylindrical region 27b: 0.16 mm), to give an external shaft having a diameter-reducing protuberance having the shape shown in Figure 11.

The external shaft was cut to make its one terminal separated by 10 mm from the diameter-reducing protuberance terminal, and proximal connecting region of the balloon was heat-fused to the cut terminal. The internal shaft was inserted from the balloon terminal, into the configuration shown in Figure 8. Then, the distance between the proximal end of the projection and the distal end of the diameter-reducing protuberance was adjusted to 1.0 mm, and the internal shaft was located at the position where it sticks out by 2 mm to the distal side of the balloon distal connecting region. The balloon distal end connecting region and the internal shaft were adhered to each other with a two-liquid urethane adhesive (Nippollan 4235, Coronate 4403, Nippon Polyurethane Industry Co., Ltd.). The shaft was cut at the position 360 mm outside the heat-fused region between the balloon and the proximal connecting region of the external shaft, and a cut of about half circle in the peripheral direction was formed on the external shaft at the position 260 mm from the heat-fused region. The internal shaft was exposed, approximately by 1 mm, out of the cut from the external shaft, and the internal and external shafts were adhered to each other with the adhesive in the state, and proximal end-side opening was formed in the guide wire lumen, to give a distal shaft.

The proximal shaft (internal diameter: 0.50 mm, external diameter: 0.66 mm, length: 1,200 mm) was made of SUS316L alloy. One end of the proximal shaft was inserted from the proximal end of the distal shaft, and the terminal of the inserted proximal shaft was brought close to the terminal of the proximal end-sided opening of guide wire lumen. The proximal shaft and the distal shaft were bonded to each other with the adhesive in that state.

A hub was prepared with polycarbonate (Makloron2658, Bayer) by injection molding. Separately, a strain relief was prepared with a polyamide elastomer (PEBAX5533SA01, Elf Atochem) by injection molding. The strain relief and the hub thus prepared were connected to the proximal end of the proximal shaft, to give an example of the balloon catheter according to the present invention.

### (Example 2)

A tube having the crosssectional shape shown in Figure 15 (diameter of inflation lumen 2a: 0.86 mm, thickness: 0.14 mm, diameter of each inflation lumen 2b: 0.06 mm) was prepared with a polyamide elastomer (PEBAX7233SA01, Elf Atochem) by extrusion molding. A balloon catheter was prepared in a similar manner to Example 1, except that the tube prepared was cut into pieces of 5 mm in length, and a tube prepared with a polyamide elastomer (PEBAX7233SA01, Elf Atochem) by extrusion molding (internal diameter 0.72 mm, external diameter 0.86 mm) was connected to both ends thereof by heat fusion to form a diameter-reducing protuberance.

### (Example 3)

A balloon catheter was prepared in a similar manner to Example 1, except that the core material 26B having the shape shown in Figure 22 (diameter of large cylindrical region 27C: 0.41 mm, diameter of small cylindrical region 27d: 0.16 mm) was used in preparing the diameter-reducing protuberance.

### (Example 4)

A tube (internal diameter: 0.43 mm, external diameter: 0.56 mm) was prepared with high-density polyethylene (HY540, Japan Polychem Corporation) by extrusion molding. The high-density polyethylene tube was cut into pieces of 298 mm in length. An X-ray impermeable marker of a platinum-tungsten alloy (tungsten content 8 wt %) (external diameter: 0.62 mm, internal diameter: 0.58 mm, length: 1.5 mm) was inserted from the terminal of the tube and bonded at the position 44 mm from the terminal by the adhesion.

A tube having the crosssectional shape shown in Figure 14 (diameter of inflation lumen 2a: 0.86 mm, thickness: 0.14 mm, diameter of each inflation lumen 2b:0.06 mm) was prepared with a polyamide elastomer (PEBAX7233SA01, Elf Atochem) by extrusion molding. A balloon catheter was prepared in a similar manner to Example 2, except that the tube prepared was cut into pieces of 5 mm in length, and a tube prepared with a polyamide elastomer (PEBAX7233SA01, ElfAtochem) by extrusion molding (internal diameter: 0.72 mm, external diameter: 0.86 mm) was heat-fused to the both ends to give a diameter-reducing protuberance.

### (Example 5)

A penetrating catheter according to the present invention was prepared in a manner similar to Example 1, except that a tube prepared with a polyamide elastomer (PEBAX6333SA01, Elf Atochem) (internal diameter: 0.72 mm, external diameter: 0.86 mm, length: 40 mm) was used, replacing the balloon.

### (Example 6)

Another penetrating catheter according to the present invention was prepared in a manner similar to Example 1, except that a tube prepared with a polyamide elastomer (PEBAX6333SA01, Elf Atochem) (internal diameter: 0.72 mm, external diameter: 0.86 mm, length: 40 mm) carrying five 100-µm holes made by excimer laser was used, replacing the balloon. The holes were placed helically clockwise toward the distal side of the catheter, on concentric circles at a gap in the axial direction of 5 mm and at the number of the holes on the same circle of 1. The phase difference between the neighboring holes was 90°.

### (Comparative Example 1)

A balloon catheter of Comparative Example 1 was prepared in a similar manner to Example 1, except that the projection and the diameter-reducing protuberance were not formed.

### (Comparative Example 2)

A penetrating catheter of Comparative Example 2 was prepared in a similar manner to Example 5, except that the projection and the diameter-reducing protuberance were not formed.

### (Comparative Example 3)

A penetrating catheter of Comparative Example 3 was prepared in a similar manner to Example 6, except that the projection and the diameter-reducing protuberance were not formed.

### (Evaluation)

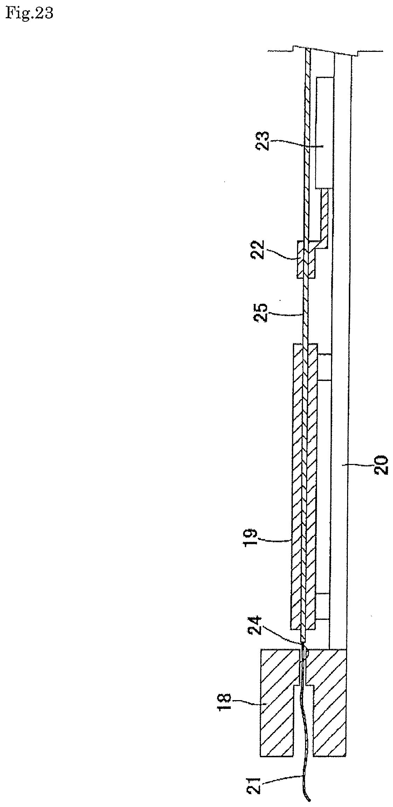

As shown in Figure 23, a catheter 25 was placed in a polyethylene simulated blood vessel 19 having an internal diameter of 3 mm and an external diameter of 5 mm fixed on a slide table 20, and the catheter 25 was connected to a force gauge 23 via a catheter-holding unit 22. The distal end of the catheter 25 is placed at the position 2 mm separated from the stainless steel simulated stricture site 18. A guide wire 21 of 0.014 inch in length is inserted previously into the guide wire lumen of the catheter 25, the guide wire 21 positioning to the distal side of catheter 25 distal end was placed in a hole 24 having a diameter of 0.45 mm formed in the simulated stricture site 18. The position of the guide wire distal end was 50 mm to the distal side of the catheter distal end. In evaluation, the force gauge 23 holding the catheter 25 is forced to move in the direction toward the stainless steel simulated stricture site 18 fixed on the slide table 20. The force gauge 23 and also the catheter 25 and the catheter-holding unit 22 moves in the direction toward the simulated stricture site 18 by the operation. The force gauge 23 was then forced to move in the direction toward the simulated stricture site 18 at a velocity of 0.5 mm /sec, until the load reaches 2.5 N.

The catheters according to the present invention obtained in Examples 1 to 4 did not show any balloon deformation in the accordion shape even at a load of 2.5N. In addition, the catheters of Examples 5 and 6 having no balloon showed favorable pressure transmission efficiency without deformation of the internal and external shafts in the nested pattern. The catheters obtained in Examples 1 to 4 did not show any strong resistance, when pulled through the hole 24, stricture site, in the evaluation system shown in Figure 23, and thus, were superior in travelling efficiency. In addition, the distal region of each of the catheters obtained in Examples 1 to 4 was flexible, and the enlargement-contraction response of the balloon was also favorable.

On the other hand, in Comparative Example 1, the balloon showed accordion-like deformation at a load of 1.3 N and did not transmit a load higher than that. With the catheters in Comparative Examples 2 and 3 having no balloon, the internal and external shafts deformed in the nested pattern at a load of 1.8 N, and the catheters did not transmit a load higher than that.

### Industrial Applicability

The catheter according to present invention is used favorably in medical application, in particular as a balloon catheter for use in peripheral angioplasty and percutaneous transluminal angioplasty (PTA, or percutaneous transluminal coronary angioplasty, PTCA) during coronary angioplasty, valve repair, or the like, a penetrating catheter for penetration through a stricture site, an injection catheter allowing administration of a treatment substance to local site, and the like.

## Claims

1. A catheter (25), comprising:
proximal (7) and distal (6) shafts respectively having proximal and distal end regions, wherein the distal end of the proximal shaft (7) and the proximal end of the distal shaft (6) are connected to each other, the proximal end of the proximal shaft (7) is connected to a hub (3) for holding the catheter (25),
at least part of the distal shaft (6) has an internal shaft (8) and an external shaft (9) enclosing the internal shaft (8) coaxially,
the internal shaft (8) extends out of the external shaft (9) toward the distal side,
the lumen of the internal shaft forms a guide wire lumen,
a projection (12) formed on on external surface of said internal shaft (8) and a diameter-reducing protuberance (13) formed on an internal surface of said external shaft (9) are in contact with each other, so as to prohibit the relative movement of the external (9) and internal (8) shafts when an insertion force at a particular intensity or more is applied to the catheter,
said projection is placed to the distal side of said diameter-reducing protuberance (13),
**characterized in that**
said projection (12) and diameter-reducing protuberance (13) are provided at the proximal end of a balloon-expandable region.

2. The catheter according to Claim 1,
further comprising a foldable balloon (5) with a proximal end region and distal end region, wherein:
said proximal end region of said balloon (5) is connected to the distal region of said external shaft (9);
said distal end region of said balloon (5) is connected to the distal region of said internal shaft (8) ; and
said projection (12) and said diameter-reducing protuberance (13) are places near said proximal end region of said balloon (5).

3. The catheter according to Claim 2,
wherein an inflation lumen (2) having a circular crosssectional shape is formed between the internal (8) and external shafts (9), and said inflation lumen (2) communicates with the space in said balloon (5).

4. The catheter according to Claim 3,
wherein said inflation lumen (2) is preserved securely in said contact region in the state where said projection (12) and said diameter-reducing protuberance (13) are in contact with each other and relative movement of said external shaft (9) and said internal shaft (8) is restricted.

## Patentansprüche

1. Katheter (25), umfassend:
proximale (7) und distale (6) Schafts, die jeweils proximale und distale Endbereiche aufweisen, wobei das distale Ende des proximalen Schafts (7) und das proximale Ende des distalen Schafts (6) miteinander verbunden sind, das proximale Ende des proximalen Schafts (7) mit einem Verbindungsstück (3) zum Halten des Katheters (25) verbunden ist,
mindestens ein Teil des distalen Schafts (6) einen internen Schaft (8) und einen externen Schaft (9), der den internen Schaft (8) koaxial umschließt, aufweist, der interne Schaft (8) aus dem externen Schaft (9) zur distalen Seite hinausragt,
das Lumen des internen Schafts ein Führungsdrahtlumen bildet.
ein Überstand (12), der sich auf der externen Oberfläche des internen Schafts (8) befindet, und ein Durchmesser-reduzierender Vorsprung (13), der sich auf einer internen Oberfläche des vorgenannten externen Schafts (9) befindet, in Kontakt miteinander stehen, so dass eine relative Bewegung des externen (9) und des internen (8) Schafts verhindert wird, wenn eine Einführkraft einer bestimmten Intensität oder mehr auf den Katheter einwirkt,
der vorgenannte Überstand zur distalen Seite des Durchmesser-reduzierenden Vorsprungs (13) angeordnet ist,
**dadurch gekennzeichnet, dass**
der vorgenannte Überstand (12) und der Durchmesser-reduzierende Vorsprung (13) am proximalen Ende des Ballon-aufweitbaren Bereichs angebracht sind.

2. Der Katheter nach Anspruch 1,
welches zusätzlich einen faltbaren Ballon (5) mit einem proximalen Endbereich und einem distalen Endbereich umfasst, wobei:
der vorgenannte proximale Endbereich des vorgenannten Ballons (5) mit dem distalen Bereich des vorgenannten externen Schafts (9) verbunden ist;
der vorgenannte distale Endbereich des vorgenannten Ballons (5) mit dem distalen Bereich des vorgenannten internen Schafts (8) verbunden ist, und
der vorgenannte Überstand (12) und der Durchmesser-reduzierende Vorsprung (13) in der Nähe des vorgenannten Endbereichs des vorgenannten Ballons (5) angeordnet sind.

3. Der Katheter nach Anspruch 2,
wobei sich ein Aufblaslumen (2) mit einer kreisförmigen Querschnittsform zwischen den internen (8) und externen Schafts (9) befindet, und das vorgenannte Aufblaslumen (2) mit dem Raum im vorgenannten Ballon (5) kommuniziert.

4. Der Katheter nach Anspruch 3,
wobei das vorgenannte Aufblaslumen (2) sicher in dem vorgenannten Kontaktbereich in dem Zustand gehalten wird, in dem der vorgenannte Überstand (12) und der Durchmesser-reduzierende Vorsprung (13) in Kontakt miteinander stehen und eine relative Bewegung des vorgenannten externen Schafts (9) und des vorgenannten internen Schafts (8) verhindert wird.

## Revendications

1. Cathéter (25), comprenant :
des tiges proximale (7) et distale (6) ayant respectivement des régions d'extrémités proximale et distale, où l'extrémité distale de la tige proximale (7) et l'extrémité proximale de la tige distale (6) sont reliées l'une à l'autre, l'extrémité proximale de la tige proximale (7) est reliée à un raccord (3) pour maintenir le cathéter (25),
au moins une partie de la tige distale (6) a une tige interne (8) et une tige externe (9) entourant la tige interne (8) de manière coaxiale,
la tige interne (8) s'étend hors de la tige externe (9) vers le côté distal,
la lumière de la tige interne forme une lumière de fil-guide,
une saillie (12) formée sur une surface externe de ladite tige interne (8) et une protubérance de réduction de diamètre (13) formée sur une surface interne de ladite tige externe (9) sont en contact l'une avec l'autre, de manière à empêcher le mouvement relatif des tiges externe (9) et interne (8) lorsqu'une force d'insertion à une intensité particulière ou plus est appliquée au cathéter,
ladite saillie est placée sur le côté distal de ladite protubérance de réduction de diamètre (13),
**caractérisé en ce que**
ladite saillie (12) et ladite protubérance de réduction de diamètre (13) sont prévues au niveau de l'extrémité proximale d'une région expansible par ballonnet.

2. Cathéter selon la revendication 1,
comprenant en outre un ballonnet pliable (5) avec une région d'extrémité proximale et une région d'extrémité distale, dans lequel :
ladite région d'extrémité proximale dudit ballonnet (5) est reliée à la région distale de ladite tige externe (9) ;
ladite région d'extrémité distale dudit ballonnet (5) est reliée à la région distale de ladite tige interne (8) ; et
ladite saillie (12) et ladite protubérance de réduction de diamètre (13) sont placées à proximité de ladite région d'extrémité proximale dudit ballonnet (5).

3. Cathéter selon la revendication 2,
dans lequel une lumière de gonflage (2), dont la coupe transversale a une forme circulaire, est formée entre les tiges interne (8) et externe (9), et ladite lumière de gonflage (2) communique avec l'espace dans ledit ballonnet (5).

4. Cathéter selon la revendication 3,
dans lequel ladite lumière de gonflage (2) est conservée en toute sécurité dans ladite région de contact à l'état où ladite saillie (12) et ladite protubérance de réduction de diamètre (13) sont en contact l'une avec l'autre et un mouvement relatif de ladite tige externe (9) et de ladite tige interne (8) est limité.
